# EUROPÄISCHE PATENTSCHRIFT

(11) **EP 2 265 245 B1**
(45) Veröffentlichungstag und Bekanntmachung des Hinweises auf die Patenterteilung: **28.04.2021**
(21) Anmeldenummer: 09721259.1
(22) Anmeldetag: 04.02.2009
(51) Int. Cl.: A61K 8/42, A61K 8/49, A61Q 15/00, A61Q 19/00, A61P 17/02, A61P 17/16

(54) **KÜHLENDE KOSMETISCHE ODER DERMATOLOGISCHE ZUBEREITUNGEN MIT EINEM GEHALT AN (1R,2S,5R)-2-ISOPROPYL-5-METHYL-N-(2-(PYRIDYN-2-YL)ETHYL-CYCLOHEXANCARBOXAMID UND/ODER (1R,2S,5R)-N-(4-(CYANOMETHYL)PHENYL)-2-ISOPROPYL-5- METHYLCYCLOHEXANCARBOXAMID ZU REDUKTION VON HAUTRÖTUNGEN**
COOLING COSMETIC OR DERMATOLOGICAL PREPARATIONS HAVING A CONTENT OF (1R,2S,5R)-2-ISOPROPYL-5-METHYL-N-(2-(PYRIDYNE-2-YL)ETHYL-CYCLOHEXANE CARBOXAMIDE AND/OR (1R,2S,5R)-N-(4-(CYANOMETHYL)PHENYL)-2-ISPROPYL-5-METHYLCYCLOHEXANE CARBOXAMIDE FOR THE REDUCTION OF ERYTHEMA
PRÉPARATIONS COSMÉTIQUES OU DERMATOLOGIQUES RAFRAÎCHISSANTES AYANT UNE TENEUR EN (1R,2S,5R)-2-ISOPROPYL-5-MÉTHYL-N-(2-(PYRIDYN-2-YL)ÉTHYL-CYCLOHEXANCARBOXAMIDE ET/OU EN (1R,2S,5R)-N-(4-(CYANOMÉTHYL)PHÉNYL)-2-ISOPROPYL-5- MÉTHYLCYCLOHEXANCARBOXAMIDE

(30) Priorität: 20.03.2008 DE 102008015426
(43) Veröffentlichungstag der Anmeldung: 29.12.2010
(73) Patentinhaber: Beiersdorf AG, 20253 Hamburg (DE)
(72) Erfinder: KOLBE, Ludger, 21255 Dohren (DE); ECKERT, Julia, 20249 Hamburg (DE); NEUFANG, Gitta, 20149 Hamburg (DE); KNAUPMEIER, Stefanie, 32108 Bad Salzuflen (DE); HÖLTKEMEIER, Stefanie, 22391 Hamburg (DE)
(74) Vertreter: Hartmann, Jost
(86) Internationale Anmeldenummer: PCT/EP2009/000717
(87) Internationale Veröffentlichungsnummer: WO 2009/115163

(56) Entgegenhaltungen:
- WO-A-93/23005
- WO-A-2005/049553
- WO-A-2006/128622
- WO-A-2007/019719
- WO-A2-2006/103401
- US-A- 4 153 679
- US-A1- 2003 235 545
- US-A1- 2005 187 211

## Beschreibung

Die vorliegende Erfindung betrifft kühlende kosmetische und dermatologische Zubereitungen zur Reduzierung von Hautrötungen nach der Rasur, insbesondere hautpflegende kosmetische und dermatologische Zubereitungen.

Als Erythem bezeichnet man eine mit dem bloßen Auge erkennbare Rötung der Haut. Sie wird durch eine gesteigerte lokale Durchblutung (Hyperämie) des Hautgewebes, zum Beispiel im Rahmen einer Entzündung, hervorgerufen. Erythematöse Hauterscheinungen können insbesondere durch die mechanische Belastung bei der Rasur auftreten.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Es war also die Aufgabe der vorliegenden Erfindung, den Nachteilen des Standes der Technik abzuhelfen.

Insbesondere sollten Wirkstoffe und Zubereitungen, solche Wirkstoffe enthaltend, zur kosmetischen und dermatologischen Behandlung und/oder Prophylaxe erythematöser, entzündlicher, allergischer oder autoimmunreaktiver Erscheinungen, insbesondere Dermatosen, aber auch des Erscheinungsbildes des "Stingings" zur Verfügung gestellt werden.

Bisher gibt es verschiedene Coolingsubstanzen, die auf der Haut ein angenehmes Frischegefühl hervorrufen. So wird beispielsweise Menthol eingesetzt, um in After Shave Formulierungen ein Frischegefühl zu bewirken. Menthol besitzt jedoch schleimhautreizende Eigenschaften und kann weitere Hautirritationen verursachen, was zu einem Brennen der Haut führt. Darüber hinaus besitzt Menthol einen starken Eigengeruch und kann zu Unverträglichkeiten im Augenbereich führen.

Überraschenderweise besitzen folgende Strukturen eine kühlende und antiinflammatorische, rötungsreduzierende Wirksamkeit:
(1*R*,2*S*,5*R*)-2-isopropyl-5-methyl-*N*-(2-(pyridin-2-yl)ethyl)-cyclohexancarboxamid 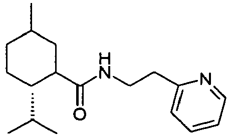
(1*R*,2*S*,5*R*)-*N*-(4-(cyanomethyl)-phenyl)-2-isopropyl-5-methylcyclohexancarboxamid 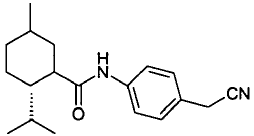 2-lsopropyl-5-methyl-cyclohexanecarbonyl-amido-propionsäure-methylester

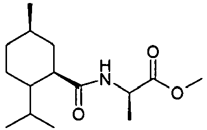

Diese rötungsreduzierenden Coolingsubstanzen sind im Stand der Technik beschrieben (WO 2007/019719 A1, WO 2005/049553 A1 und WO 2006/103401 A1). Sie können alleine oder in Kombination mit anderen Coolingsubstanzen eingesetzt werden.

Beispiele für andere Coolingsubstanzen sind Menthol, Menthoxypropanediol (Cooling Agent 10), Menthone, Ethyl Menthane Carboxamido acetate (WS-5), Isopulegol (Coolact P), Menthanediol (Coolact 38D), N,2,3-trimethyl-2-isopropylbutanamide (WS-23), Ethyl Menthane Carboxamide (WS-3), menthone glycerine acetal (Frescolat MGA) oder mono-menthyl succinate (Physcool).

Es hat nicht an Versuchen gefehlt, Hautrötungen nach der Rasur zu verringern und Rasurbrand vorzubeugen.

Es war eine Aufgabe der vorliegenden Erfindung, den Übelständen des Standes der Technik abzuhelfen und erythemreduzierende Zubereitungen zur Verfügung zu stellen, welche einfach herzustellen sind, keine Reizwirkung auf Haut oder Schleimhäute ausüben, geruchsneutral sind sowie bei bestimmungsgemäßer Anwendung zusätzlich eine angenehme Kühlung spenden
Diese Aufgabe wurde erfindungsgemäß gelöst durch Wirkstoffkombinationen aus (1 R,2S,5R)-2-Isopropyl-5-methyl-N-(2-(pyridin-2-yl)-ethyl-cyclohexancarboxamid und/oder (1 R,2S,5R)-N-(4-(Cyanomethyl)-phenyl)-2- isopropyl-5-methylcyclohexancarboxamid und 2-Isopropyl-5-methyl-cyclohexancarbonyl-amido-propionsäure-methylester zur Verwendung gegen Hautrötung in kosmetischen oder dermatologischen Zubereitungen,dadurch gekennzeichnet, dass die Zubereitungen eine oder mehrere Substanzen gewählt aus der Gruppe Lichtschutzmittel, Antitranspirantien, Deodorantien enthalten.

Außerdem erfindungsgemäß sind Zubereitungen enthaltend Wirkstoffkombinationen wie vorhergehend beschrieben, dadurch gekennzeichnet, dass sie 0,001 - 10 Gew.-%, besonders bevorzugt 0,01 - 1 Gew.-%, an (1R,2S,5R)-2-Isopropyl-5-methyl-N-(2-(pyridin-2-yl)ethyl-cyclohexancarboxamid und/oder (1R,2S,5R)-N-(4-(Cyanomethyl)-phenyl)-2-isopropyl-5-methylcyclohexancarboxamid enthalten, bezogen auf die Gesamtzusammensetzung der Zubereitungen. Diese Zubereitungen sind bevorzugt dadurch gekennzeichnet, dass sie 0,001 - 10 Gew.- %, besonders bevorzugt 0,01 - 1 Gew.-%, an 2-Isopropyl-5-methyl-cyclohexanecarbonyl-amido-propionsäure-methylester enthalten. Es war erstaunlich und für den Fachmann nicht vorhersehbar, dass topische kosmetische oder dermatologische Zubereitungen zur Verringerung von Hautrötungen nach der Rasur mit einem Gehalt an (1R,2S,5R)-2-Isopropyl-5-methyl-N-(2-(pyridyn-2-yl)ethyl-cyclohexancarboxamid und/oder (1R,2S,5R)-N-(4-(Cyanomethyl)-phenyl)-2-isopropyl-5-methylcyclohexancarboxamid und 2-Isopropyl-5-methyl-cyclohexanecarbonyl-amido-propionsäure-methylester die Nachteile des Standes der Technik beseitigen.

Die Zubereitungen sind einfach zu formulieren und stellen keine großen Anforderungen an Herstellungsvorgänge.

Bevorzugt ist die Herstellung einer O/W Emulsion. Die Cooling Substanzen werden in Polyolen vorgelöst und bei ca. 40°C zur Emulsion vor dem Abkühlen auf Raumtemperatur zugegeben.

Die Erfindung umfasst auch die Verwendung von (1R,2S,5R)-2-Isopropyl-5-methyl-N-(2-(pyridin-2-yl)ethyl-cyclohexancarboxamid und/oder (1R,2S,5R)-N-(4-(Cyanomethyl)-phenyl)-2-isopropyl-5-methylcyclohexancarboxamid in After Shave Formulierungen zur Verringerung von Hautrötungen und die Verwendung von (1R,2S,5R)-2-Isopropyl-5-methyl-N-(2-(pyridin-2-yl)ethyl-cyclohexancarboxamid und/oder (1R,2S,5R)-N-(4-(Cyanomethyl)-phenyl)-2-isopropyl-5-methylcyclohexancarboxamid in Deo Formulierungen zur Verringerung von Hautrötungen. Dabei ist erfindungsgemäß zusätzlich 2-Isopropyl-5-methyl-cyclohexanecarbonyl-amido-propionsäure-methylester enthalten. Besonders bevorzugt ist es, wenn die Zubereitungen 0,001 - 10 Gew.-%, besonders bevorzugt 0,01 - 1 Gew.-%, an (1R,2S,5R)-2-Isopropyl-5-methyl-N-(2-(pyridin-2-yl)ethyl-cyclohexancarboxamid und/oder (1R,2S,5R)-N-(4-(Cyanomethyl)-phenyl)-2-isopropyl-5-methylcyclohexancarboxamid enthalten, bezogen auf die Gesamtzusammensetzung der Zubereitungen. Besonders bevorzugt ist es, wenn die Zubereitungen 0,001 - 10 Gew.-%, besonders bevorzugt 0,01 - 1 Gew.-%, an 2-Isopropyl-5-methyl-cyclohexanecarbonyl-amido-propionsäure-methylester enthalten. Weiter ist es bevorzugt, wenn die Zubereitungen Lichtschutzmittel enthalten. Weiter ist es bevorzugt, wenn die Zubereitungen Antioxidantien enthalten. Weiter ist es bevorzugt, wenn die Zubereitungen Vitamine enthalten. Weiter ist es bevorzugt, wenn die Zubereitungen Antitranspirantien enthalten. Weiter ist es bevorzugt, wenn die Zubereitungen Deodorantien enthalten.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an üblichen Antioxidantien ist im Allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wässrige Lösungen
- Öle, wie Triglyceride der Caprin- oder der Caprylsäure, vorzugsweise aber Sonnenblumenöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder - monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder - distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die erfindungsgemäße Zusammensetzung soll in einer für die topische Applikation geeigneten Form vorliegen. Beispielsweise kann die Zusammensetzung in Form einer Creme, einer Lotion, eines Gels, einer Salbe, einer Tinktur, eines Hautöls, einer Milch, eines Balsams, eines Tensidschaumes, eines Emulsionsschaumes, eines Tensidgeles, eines mit der Zusammensetzung imprägnierten Pflasters, eines mit der Zusammensetzung imprägnierten Tuchs, einer mit der Zusammensetzung imprägniertenTextilie, eines mit der Zusammensetzung imprägnierten Pads, eines Sprays, eines Aerosols, eines Roll-on, eines Stifts, einer Soft Solid, eines Puders oder eines Pudersprays vorliegen

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und dermatologische Anwendungen geeigneten oder gebräuchlichen Antioxidantien verwendet werden.

Ganz besonders vorteilhaft im Sinne der vorliegenden Erfindung können wasserlösliche Antioxidantien eingesetzt werden, wie beispielsweise Vitamine, z. B. Ascorbinsäure und deren Derivate - insbesondere Ascorbylpalmitat, Na- und Mg-Ascorbylphosphat und Ascorbylacetat - sowie Rutinsäure und deren Derivate insbesondere alpha Glucosylrutin, Quercetin und Isoquercetin, außerdem Isoflavone, insbesondere Genistein, Genistin, Daidzein.

Besonders bevorzugte Antioxidantien sind ferner Vitamin E und dessen Derivate (insbesondere Vitamin E-Acetat), Vitamin A und dessen Derivate (insbesondere Vitamin-A-Palmitat) sowie Carnosin, Butylhydroxytoluol, Butylhydroxyanisol Ferulasäure, ungesättigte Fettsäuren und deren Derivate (z.B. gamma-Linolensäure, Linolsäure, Ölsäure), Folsäure und deren Derivate, beta-Alanin sowie Carotinoide (insbesondere beta-Carotin) und Phytoen.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wässrigen Phase einzusetzen.

Vorteilhaft können die Lichtschutzformulierungen erfindungsgemäß weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen Dabei ist Beispiel 1 erfindungsgemäß, die anderen Beispiele gelten als Referenzen.

**O/W-Nachtcreme**

| **Beispiel** | **1** |
|---|---|
| Glycerylstearatcitrat | 2 |
| Sheabutter | 2 |
| Stearylalkohol | 2 |
| Cetylalkohol | 2 |
| Hydrierte Kokosfettglyceride (Hydrogenated Coco Glycerides) | 2 |
| Caprylsäure/Caprinsäure Triglycerid | 2 |
| Ethylhexylkokosfettsäureester | 2 |
| Cyclometicone | 3 |
| Dicaprylylether | 2 |
| Tocopherol | 0,1 |
| Folsäure | 0,1 |
| (1 R,2S,5R)-2-Isopropyl-5-methyl-N-(2-(pyridin-2-yl)ethyl-cyclohexancarboxamid | 0.3 |
| Retinylpalmitat | 0,1 |
| Phenoxyethanol | 0,6 |
| p-Hydroxybenzoesäu realkylester (Para ben) | 0,6 |
| Ethylhexylglycerin | 0,5 |
| Polyacrylsäure (Carbomer) | 0,1 |
| EDTA | 0,2 |
| Glycerin | 10 |
| wasser- und/oder öllösliche Farbstoffe | 0,05 |
| Füllstoffe/ Additive (SiO₂, BHT) | 0,2 |
| 2-Isopropyl-5-methyl-cyclohexanecarbonyl-amidopropionsäure-methylester | 0.1 |
| Wasser | ad 100 |

**Hautpflegecreme**

| | **Gew.-%** | **Gew.-%** |
|---|---|---|
| Glycerylstearat, selbstemulgierend | 6 | 5 |
| Stearylalkohol | 1 | 1 |
| Sheabutter | 1 | 1 |
| C12-15 Alkylbenzoat | 3 | 3 |
| Caprylsäure/Caprinsäure Triglycerid | 2 | 2 |
| Mineralöl | 1 | 1 |
| Dicarprylylcarbonat | 3 | 3 |
| Ethylhexylcyanodiphenylacrylat (Octocrylen) | 5 | 5 |
| Ethylhexyltriazon | 1 | 1 |
| Bis-Ethylhexyloxyphenolmethoxyphenyltriazin | 2 | 2 |
| Citronensäure, Natriumsalz | 0.1 | 0.1 |
| Silymarin | | 0,05 |
| Phenoxyethanol | 0,6 | 0,6 |
| p-Hydroxybenzoesäurealkylester (Paraben) | 0,3 | 0,3 |
| Hexamidindiisethionat | 0,04 | 0,04 |
| 1,3-Dimethylol-5,5-dimethylhydantoin(DMDM Hydantoin) | 0,1 | 0,1 |
| EDTA | 0,2 | 0,2 |
| Vitamin B3 (Niacinamid) | 0,2 | |
| Ammoniumacryloyldimethyltaurate/ Vinylpyrrolidoncopolymere | 0,5 | 0,3 |
| Glycerin | 10 | 10 |
| (1 R,2S,5R)-N-(4-(Cyanomethyl)-phenyl)-2-isopropyl-5-methylcyclohexancarboxamid | 1 | 0,3 |
| Additive (Distärkephosphat, SiO₂, BHT) | 1 | 1 |
| Parfüm | q.s. | q.s. |
| Wasser | ad 100 | ad 100 |

**Sonnenschutzcreme**

| | **Gew.-%** | **Gew.-%** |
|---|---|---|
| Glycerylstearat | 3 | 3 |
| PEG-40-Stearat | 1 | 1 |
| Cetearylalkohol | 3 | 1 |
| Sheabutter | 2 | 2 |
| C12-15 Alkylbenzoat | 2 | 2 |
| Cocosglyceride | - | 2 |
| Octyldodecanol | 3 | 3 |
| Bienenwachs | 1 | - |
| (1 R,2S,5R)-N-(4-(Cyanomethyl)-phenyl)-2-isopropyl-5-methylcyclohexancarboxamid | 0,2 | - |
| Ethylhexylmethoxycinnamat | 5 | 5 |
| Phenylbenzimidazol sulfonsäure | 2 | 2 |
| Butylmethoxydibenzoylmethan | 2 | 2 |
| TiO2 | 2 | 2 |
| Natriumascorbylphosphat | 0,1 | 0,1 |
| Tocopherylacetat | 1 | 1 |
| Methylpropandiol | 3 | 3 |
| (1 R,2S,5R)-2-Isopropyl-5-methyl-N-(2-(pyridin-2-yl)ethyl-cyclohexancarboxamid | 0,1 | 0,5 |
| p-Hydroxybenzoesäurealkylester (Paraben) | 0,2 | 0,2 |
| EDTA | 0,2 | 0,2 |
| Diazolidinylharnstoff | 0,1 | 0,1 |
| Carbomer | 0,1 | 0,1 |
| Carrageenan | 0,1 | 0,1 |
| Glycerin | 7 | 7 |
| Additive (BHT, Nylon-6) | 0,4 | 0,4 |
| Parfüm | q.s. | q.s. |
| Wasser | ad 100 | ad 100 |

**Hautrötungsminimierende O/W-Creme**

| | **Gew.-%** |
|---|---|
| Glycerylstearat | 1 |
| Stearinsäure | 3 |
| Stearylalkohol | 2 |
| Cetylalkohol | 2 |
| C12-15 Alkylbenzoat | 2 |
| Caprylsäure/Caprinsäure Triglycerid | 2 |
| Macadamiaöl | 1 |
| Myristylmyristate | 2 |
| Dimethicone | 2 |
| Hydrierte Kokosfettglyceride (Hydrogenated Coco Glycerides) | 1 |
| Ethylhexylglycerin | 0,5 |
| Tocopherylacetat | 1 |
| (1 R,2S,5R)-N-(4-(Cyanomethyl)-phenyl)-2-isopropyl-5-methylcyclohexancarboxamid | 0,1 |
| Kreatin | 0,1 |
| Ubichinon (Q10) | 0,03 |
| Phenoxyethanol | 0,4 |
| p-Hydroxybenzoesäurealkylester (Paraben) | 0,3 |
| lodopropinylbutylcarbamat | 0,02 |
| Cyclodextrin | 0,3 |
| Natrium citrat | 0,2 |
| Carbomer | 0,3 |
| Glycerin | 5 |
| Methylpropandiol | 3 |
| Additive (SiO₂, Talkum) | 0,5 |
| Parfüm | q.s. |
| Wasser | ad 100 |

**After Sun Gel**

| | **Gew.-%** |
|---|---|
| Cetylalkohol | 2 |
| Sheabutter | 1 |
| Caprylsäure/Caprinsäure Triglycerid | 2 |
| Octyldodecanol | 1 |
| Dicaprylylcarbonat | 5 |
| Dimethicon | 2 |
| Polydecen | 2 |
| Methylpalmitat | 3 |
| Licochalcone A | 0,02 |
| Natriumascorbylphosphat | 0,05 |
| EDTA | 0,2 |
| (1 R,2S,5R)-2-Isopropyl-5-methyl-N-(2-(pyridin-2-yl)ethyl-cyclohexancarboxamid | 1.5 |
| Phenoxyethanol | 0,3 |
| p-Hydroxybenzoesäurealkylester (Paraben) | 0,4 |
| Vernetztes Alkylacrylat (Alkylacrylate Crosspolymer) | 0,2 |
| Glycerin | 5 |
| Parfüm | q.s. |
| Wasser | ad 100 |

**Hautberuhigender After Shave Balm**

| | Gew.-% | Gew.-% |
|---|---|---|
| Triceteareth-4-Phsophate | 0,5 | 1,0 |
| Cyclometicone | 2,0 | 2,0 |
| Octyldodecanol | - | 1,0 |
| Dicaprylylcarbonat | 3,0 | 3,0 |
| Methylpalmitat | 2,0 | - |
| Carnitin | | 0,2 |
| Macadamiaöl | 0,2 | 0,1 |
| (1 R,2S,5R)-2-Isopropyl-5-methyl-N-(2-(pyridyn-2-yl)ethyl-cyclohexancarboxamid | 0,15 | |
| Ascorylphosphat | | 0,2 |
| Arginin | 0,2 | |
| Phenoxyethanol | 0,5 | 0,5 |
| p-Hydroxybenzoesäurealkylester (Paraben) | 0,4 | 0,4 |
| Vernetztes Alkylacrylat (Alkylacrylate Crosspolymer) | 0,3 | 0,3 |
| Distärkephosphat | 1,0 | 1,0 |
| Xanthan Gum | 0,1 | 0,1 |
| (1 R,2S,5R)-N-(4-(Cyanomethyl)-phenyl)-2-isopropyl-5-methylcyclohexancarboxamid | | 0,2 |
| Glycerin | 4,0 | 4,0 |
| Parfüm | q.s. | q.s. |
| Wasser | ad 100 | ad 100 |

**(selbstschäumender) hautberuhigender Rasierschaum**

| | **Gew.-%** |
|---|---|
| Stearinsäure | 6 |
| Laureth-23 | 4 |
| Stearylalkohol | 0,5 |
| Sojaöl | 0,2 |
| Avocadoöl | 0,1 |
| PEG-12-Dimeticone | 0,8 |
| Hydroxypropylmethylcellulose | 0,4 |
| PEG-7M | 0,2 |
| Natriu mascorbylphosphat | 0,05 |
| Additive (EDTA, BHT, Silica | 0,2 |
| (1 R,2S,5R)-2-Isopropyl-5-methyl-N-(2-(pyridin-2-yl)ethyl-cyclohexancarboxamid | 0,1 |
| (1R,2S,5R)-N-(4-(Cyanomethyl)-phenyl)-2-isopropyl-5-methylcyclohexancarboxamid | 0,05 |
| p-Hydroxybenzoesäurealkylester (Paraben) | 0,4 |
| Glycerin | 3 |
| Treibgas: Isobutan, Propan, Butan | qs |
| Parfüm | q.s. |
| Wasser | ad 100 |

| | |
|---|---|
| Packmittel: Aerosolbehälter | |

**Deodorant Roll-on**

| **Rohstoff (INCI)** | **Gew.-%** | | | |
|---|---|---|---|---|
| Polyethylenglykol(21)stearylether | 3,000 | 3,000 | 3,000 | 1,500 |
| Polyethylenglykol(2)stearylether | 2,000 | 2,000 | 2,500 | 3,000 |
| Polypropylenglykol(15)stearylether | 2,000 | 3,000 | 3,000 | |
| Cocosnussfettsäure-2-ethylhexylester | - | - | 1,000 | - |
| EDTA | 0,200 | 0,200 | 0,200 | 0,200 |
| Avocadoöl | 0,100 | 0,100 | 0,100 | 0,100 |
| Aluminiumchlorohydrat | 8,000 | 12,000 | 10,000 | 5,000 |
| Parfüm, Antioxidantien (BHT, Ascorbylpalmitat) | q.s. | q.s. | q.s. | q.s. |
| (1R,2S,5R)-2-lsopropyl-5-methyl-N-(2-(pyridin-2-yl)ethyl-cyclohexancarboxamid | 0,1 | - | 0,1 | - |
| (1R,2S,5R)-N-(4-(Cyanomethyl)-phenyl)-2-isopropyl-5-methylcyclohexancarboxamid | - | 0,2 | 0,05 | 0,1 |
| Wasser, ad | 100,000 | 100,000 | 100,000 | 100,00 |

## Patentansprüche

1. Wirkstoffkombinationen aus (1R,2S,5R)-2-Isopropyl-5-methyl-N-(2-(pyridin-2-yl)-ethyl-cyclohexancarboxamid und/oder (1R,2S,5R)-N-(4-(Cyanomethyl)-phenyl)-2-isopropyl-5-methylcyclohexancarboxamid und 2-Isopropyl-5-methyl-cyclohexancarbonyl-amido-propionsäure-methylester zur Verwendung gegen Hautrötung in kosmetischen oder dermatologischen Zubereitungen, **dadurch gekennzeichnet, dass** die Zubereitungen eine oder mehrere Substanzen gewählt aus der Gruppe Lichtschutzmittel, Antitranspirantien, Deodorantien enthalten.

2. Zubereitungen enthaltend Wirkstoffkombinationen nach Anspruch 1, **dadurch gekennzeichnet, dass** sie 0,001 - 10 Gew.-%, besonders bevorzugt 0,01 - 1 Gew.-%, an (1R,2S,5R)-2-Isopropyl-5-methyl-N-(2-(pyridin-2-yl)ethyl-cyclohexancarboxamid und/oder (1R,2S,5R)-N-(4-(Cyanomethyl)-phenyl)-2-isopropyl-5-methylcyclohexancarboxamid enthalten, bezogen auf die Gesamtzusammensetzung der Zubereitungen.

3. Zubereitungen nach Anspruch 2 oder Wirkstoffkombinationen zur Verwendung nach Anspruch 1, **dadurch gekennzeichnet, dass** sie 0,001 - 10 Gew.- %, besonders bevorzugt 0,01 - 1 Gew.-%, an 2-Isopropyl-5-methyl-cyclohexanecarbonyl-amido-propionsäure-methylester enthalten.

## Claims

1. Active ingredient combinations of (1R,2S,5R)-2-isopropyl-5-methyl-N-(2-(pyridin-2-yl)ethylcyclohexanecarboxamide and/or (1R,2S,5R)-N-(4-(cyanomethyl)phenyl)-2-isopropyl-5-methylcyclohexanecarboxamide and methyl 2-isopropyl-5-methylcyclohex-anecarbonylamidopropionate for use against erythema in cosmetic or dermatological preparations, **characterized in that** the preparations comprise one or more substances selected from the group comprising light protection agents, antiperspirants, deodorants.

2. Preparations comprising active ingredient combinations according to Claim 1, **characterized in that** they comprise 0.001 - 10% by weight, particularly preferably 0.01 - 1% by weight of (1R,2S,5R)-2-isopropyl-5-methyl-N-(2-(pyridin-2-yl)ethylcyclohexanecar-boxamide and/or (1R,2S,5R)-N-(4-(cyanomethyl)phenyl)-2-isopropyl-5-methylcyclohexanecarboxamide, based on the total composition of the preparations.

3. Preparations according to Claim 2 or active ingredient combinations for use according to Claim 1, **characterized in that** they comprise 0.001 - 10% by weight, particularly preferably 0.01 - 1% by weight of methyl 2-isopropyl-5-methylcyclohexanecarbonyl-amidopropionate.

## Revendications

1. Combinaisons de principes actifs composées de (1R,2S,5R)-2-isopropyl-5-méthyl-N-(2-(pyridin-2-yl)-éthyl-cyclohexanecarboxamide et/ou (1R,2S,5R)-N-(4-(cyanométhyl)-phényl)-2-isopropyl-5-méthylcyclohexanecarboxamide et d'ester méthylique d'acide 2-isopropyl-5-méthyl-cyclohexanecarbonyl-amido-propionique pour une utilisation contre des rougeurs de la peau dans des préparations cosmétiques ou dermatologiques, **caractérisées en ce que** les préparations contiennent une ou plusieurs substances choisies dans le groupe des agents photoprotecteurs, des antitranspirants, des déodorants.

2. Préparations contenant des combinaisons de principes actifs selon la revendication 1, **caractérisées en ce qu'**elles contiennent 0,001 à 10 % en poids, particulièrement préférablement 0,01 à 1 % en poids, de (1R,2S,5R)-2-isopropyl-5-méthyl-N-(2-(pyridin-2-yl)-éthyl-cyclohexanecarboxamide et/ou (1R,2S,5R)-N-(4-(cyanométhyl)-phényl)-2-isopro-pyl-5-méthylcyclohexanecarboxamide, par rapport à la composition totale des préparations.

3. Préparations selon la revendication 2 ou combinaisons de principes actifs pour l'utilisation selon la revendication 1, **caractérisées en ce qu'**elles contiennent 0,001 à 10 % en poids, particulièrement préférablement 0,01 à 1 % en poids, d'ester méthylique d'acide 2-isopropyl-5-méthyl-cyclohexanecarbonyl-amido-propionique.
